(19) Europäisches Patentamt / European Patent Office / Office européen des brevets

(11) **EP 3 758 770 B1**

(12) **EUROPÄISCHE PATENTSCHRIFT**

(45) Veröffentlichungstag und Bekanntmachung des Hinweises auf die Patenterteilung:
**29.10.2025 Patentblatt 2025/44**

(21) Anmeldenummer: **19728901.0**

(22) Anmeldetag: **24.05.2019**

(51) Internationale Patentklassifikation (IPC):
**A61M 1/16** (2006.01)    **A61M 16/00** (2006.01)

(52) Gemeinsame Patentklassifikation (CPC):
(C-Sets verfügbar)
**A61M 16/00; A61M 1/1698;** A61M 2205/05;
A61M 2205/502; A61M 2230/42; A61M 2230/432
(Forts.)

(86) Internationale Anmeldenummer:
**PCT/EP2019/063442**

(87) Internationale Veröffentlichungsnummer:
**WO 2020/007530 (09.01.2020 Gazette 2020/02)**

(54) **SYSTEM ZUR UNTERSTÜTZUNG DES BLUTGASAUSTAUSCHS MITTELS BEATMUNG UND EXTRAKORPORALEM BLUTGASAUSTAUSCH UND NACH DEM VERFAHREN ARBEITENDES SYSTEM**

METHOD FOR SUPPORTING THE BLOOD GAS EXCHANGE BY MEANS OF VENTILATION AND EXTRACORPOREAL BLOOD GAS EXCHANGE, AND SYSTEM OPERATING ACCORDING TO THE METHOD

SYSTÈME DE SOUTIEN DE L'ÉCHANGE GAZEUX SANGUIN PAR LA RESPIRATION ARTIFICIELLE ET L'ÉCHANGE GAZEUX SANGUIN EXTRACORPOREL ET SYSTÈME FONCTIONNANT SELON LE PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.07.2018 DE 102018005228**

(43) Veröffentlichungstag der Anmeldung:
**06.01.2021 Patentblatt 2021/01**

(73) Patentinhaber: **Drägerwerk AG & Co. KGaA 23558 Lübeck (DE)**

(72) Erfinder:
• **KULLIK, Götz**
  **23568 Lübeck (DE)**
• **VON BLUMENTHAL, Tilman**
  **23562 Lübeck (DE)**
• **DICKS, Bernd-Michael**
  **23560 Lübeck (DE)**

(56) Entgegenhaltungen:
WO-A1-2011/021978    WO-A1-2015/185618
WO-A1-2018/106164    US-A1- 2015 034 082

(52) Gemeinsame Patentklassifikation (CPC): (Forts.)

C-Sets
A61M 2230/432, A61M 2230/005

**Beschreibung**

Die Erfindung betrifft ein System zur Unterstützung des Blutgasaustauschs mittels Beatmung und extrakorporalem Blutgasaustausch.

Sobald ein Patient nicht mehr ausreichend spontan atmen kann, wird ihm eine technische Hilfe gegeben, um den Austausch von Sauerstoff ($O_2$) und Kohlendioxid ($CO_2$) mit den Körperzellen zu ermöglichen. Dabei wird die Lunge des Patienten durch verschiedene Formen mechanisch-pneumatischer Atemhilfen (Beatmungsgerät) unterstützt und/oder das Blut durch extrakorporalen Blutgasaustausch (extrakorporale Membranoxygenierung) mit Hilfe von Membranen mit Sauerstoff angereichert und Kohlendioxid abgeführt.

Der Grad einer Invasivität der beiden Hilfsmaßnahmen kann unterschiedlich sein. Bei der Beatmung mittels Beatmungsgerät sind es Gaskonzentrationen, Drücke und Volumina, die gesteuert werden können, und damit verbunden ist die Notwendigkeit verschiedener Zugänge zur Lunge. Beispielsweise ist eine Beatmung ohne Intubation (non-invasive ventilation; NIV) nur bis zu einem bestimmten Druck möglich.

Extrakorporale Membranoxygenierung (ECMO; andere Bezeichnung: Extracorporeal Life Support = ECLS) ist aus sogenannten Herz-Lungen-Maschinen (HLM) bekannt.

Zur parallelen Verwendung zusammen mit einer Beatmung mittels Beatmungsgerät gibt es bezüglich $CO_2$-Austausch und minimalem Blutfluss optimierte extrakorporale Membran-$CO_2$-Austauscher ($ECCO_2R$).

Sie werden nach Blutflussraten unterschieden, die dann entsprechend dimensionierte blutige Zugänge erfordern; beispielsweise ist ein veno-venöser Austausch (ohne arteriellen Zugang) nur bis zu einem extrakorporalen Blutfluss bis zu maximal 700 ml/min möglich.

Aus der US 2015/034082 A1 sind ein kombiniertes System mit Beatmungsgerät und $CO_2$-Austauscher und ein Verfahren zu dessen Betrieb bekannt. Die US 2015/034082 A1 beschreibt im Detail einzelne bei einem solchen kombinierten Betrieb in Betracht zu ziehende Messwerte und deren Berücksichtigung im therapeutischen Prozess in Form von Maßnahmen bei einer Grenzwertüberschreitung.

In der EP 3 291 854 A1 ist eine tragbare Gasaustauschvorrichtung beschrieben. Die tragbare Gasaustauschvorrichtung weist Abschnitte zum Gasaustausch, eine Mischkammer, optionale Partikelfilter und gesteuerte Pumpen zur Förderung von Fluiden, wie Blut und Sauerstoff enthaltende Gasgemische, auf.

Da mit dem Grad der Invasivität das Komplikationsrisiko steigt, ist es sinnvoll, beide Methoden so zu kombinieren, dass die jeweilige Invasivität gering ist und damit auch das Gesamtkomplikationsrisiko möglichst gering ist.

Ein besonders nützliches Beispiel für die Kombination von Beatmung mittels Beatmungsgerät und extrakorporaler Membranoxygenierung mittels $CO_2$-Austauscher ist die Behandlung von Patienten mit COPD (chronic obstructive lung disease).

Bei diesen Patienten ist der Zugang zur Lunge verengt. Die ausreichende Versorgung mit Sauerstoff ($O_2$) kann bei diesen Patienten noch gut durch Gabe einer erhöhten $O_2$-Konzentration sichergestellt werden. Um jedoch Kohlendioxid ($CO_2$) allein über die Lunge in notwendigem Maße abzutransportieren, sind hohe Minutenvolumina bei der Beatmung notwendig. In Folge des hohen pneumatischen Widerstandes erfordert dies einen hohen Beatmungsdruck. Um den $CO_2$-Abtransport sicherzustellen, wird es dann notwendig, von einer Maskenbeatmung auf eine invasive Beatmung mit Tubus oder Tracheotomie umzusteigen. Die Vermeidung einer invasiven Beatmung hat aber wegen der damit verbundenen Komplikationsrisiken gerade bei COPD-Patienten eine hohe Priorität.

Die beatmungsunterstützende extrakorporale Membranoxygenierung mittels $CO_2$-Austauscher entwickelt sich zunehmend als Möglichkeit zur Vermeidung des Umstiegs auf invasive Beatmung - insbesondere in der veno-venösen Form mit geringem Blutfluss.

Bei der Parallelverwendung von Maskenbeatmung und $CO_2$-Austauscher ($ECCO_2R$) ist die Abstimmung aller Einstellparameter für eine schonende und zielführende Steuerung und Regelung zum Gesamtwohl des Patienten notwendig. Diese wird heute eher nach subjektiven Erfahrungswerten durchgeführt. Dies ist fehleranfällig. Zudem werden die Durchführung vergleichbarer Studien und die Entwicklung evidenzbasierter Therapierichtlinien erschwert.

Eine Aufgabe der vorliegenden Erfindung besteht darin, eine Möglichkeit zur Steuerung und Regelung von $CO_2$- und $O_2$-Austausch für Patienten mit nichtinvasiver Beatmung der Lunge und extrakorporalem Blutgasaustausch möglichst auf Basis von reproduzier- und vergleichbaren physiologisch relevanten Messwerten anzugeben.

Zentrales Ziel des CO2-Austausches ist ein arterieller CO2-Partialdruck, der zu einem ruhigen bestimmten Atemantrieb (Eigenatmung; Druckunterstützung durch Atemgerät, Atemgerät getriggert durch Eigenatmung) führt, also weder zu Hyper noch zu Hypoventilation. Während dies bei den meisten Patienten ein definierter Zielbereich von ca. 40 - 45 mmHG ist, ist bei COPD-Patienten die körpereigene Sensorik bereits so an einen erhöhten Partialdruck angepasst, dass der Zielbereich individuell bis zu 65 mmHG betragen kann. Dazu ist erfindungsgemäß ein $CO_2$- Austauscher vorgesehen. Als $CO_2$- Austauscher können im Sinne der vorliegenden Erfindung System zur Extrakoporalen Membranoxygnierung (ECMO) oder sogenannte Herz- Lungen- Maschinen (HLM) Verwendung finden, in denen der Gasaustausch außerhalb des menschlichen Körpers stattfindet, wobei dem Blut eines Patienten mit Hilfe einer Membran eine gewisse Menge an Sauerstoff zugeführt wird und Kohlendioxid mit Hilfe der Membran aus dem Blut des Patienten fortgeführt wird.

Problematisch ist bereits eine dafür notwendige Bereitstellung objektiver, kontinuierlicher Messwerte für zumindest einzelne relevante Transportparameter, da nicht für alle Parameter geeignete Messverfahren bekannt sind. Als Transportparameter kommen insbesondere folgende Parameter in Betracht:

| | | |
|---|---|---|
| $CaO_2$ | | arterial $O_2$ content |
| $CvO_2$ | | venous $O_2$ content |
| $CaCO_2$ | | arterial $CO_2$ content |
| $CvCO_2$ | | venous $CO_2$ content |
| $DO_2$ | | $O_2$ delivery |
| $VO_2$ | $Q \times (CaO_2 - CvO_2)$ | $O_2$ uptake |
| $VCO_2$ | $Q \times (CvCO_2 - CaCO_2)$ | $CO_2$ elimination |
| $RQ$ | $VCO_2/VO_2)$ | respiratory quotient |

Auch eine Ermittlung eines objektiv-kontinuierlichen Messwerts für den $CO_2$-Gehalt im Blut auf Basis eines messtechnisch bestimmten $CO_2$-Konzentrationsmesswertes im Atemgas ist unter Maskenbeatmung trotz optimaler Anpassung des Messaufbaus nicht mit hinreichender Genauigkeit möglich.

Vielmehr sind bisher fehlerbehaftete Schätzverfahren notwendig, um zumindest einen Näherungswert für den $CO_2$-Gehalt des Bluts zu erhalten. Zudem ist der therapeutisch anzustrebende Zielwert des $CO_2$-Gehalts des Bluts für COPD-Patienten im Vergleich zu Normalpatienten erhöht und individuell stark unterschiedlich. Ein solcher Zielwert ist daher für den Therapeuten nicht einfach zu ermitteln oder gar aus einer Guideline abzulesen.

Die hier vorgestellte Neuerung fußt auf dem Ansatz einer Ermittlung eines Trendparameters, insbesondere eines einheitenlosen Trendparameters, für den $CO_2$-Gehalt im Blut sowie der Verwendung eines solchen Trendparameters für ein objektiv vergleichbares und reproduzierbares Kontrollverfahren für den $CO_2$-Partialdruck des Bluts ($PaCO_2$) und zwar ausgehend von einem ärztlich festgestellten stabilen Zustand.

Die messtechnisch erfasste exspiratorische $CO_2$-Konzentration im Atemgas des Patienten liegt oftmals durch auch als ein exspiratorischer $CO_2$-Konzentrationsmesswert vor, welcher gegen Ende der Ausatmung (Exspiration) als sogenannte endexspiratorische $CO_2$-Konzentration im Atemgas des Patienten, oftmals auch als endtidale Kohlendioxidkonzentration (et $CO_2$) bezeichnet, erfasst wird. Für die Diagnostik hinsichtlich der Umsetzung von Sauerstoff zu Kohlendioxid im Stoffwechsel stellt die endtidale Kohlendioxidkonzentration (et $CO_2$) in vielen Fällen einen hinreichend signifikanten dar, aber auch der zeitliche Verlauf der exspiratorischen $CO_2$-Konzentration, bzw. repräsentative Werte aus diesem zeitlichen Verlauf können ergänzende Informationen zum Gasaustausch und Stoffwechsel aufweisen.

Im Sinne der folgenden Erfindung werden folglich die exspiratorischen $CO_2$-Parameter und exspiratorischen $CO_2$- Messwerte, wie auch die endexspiratorischen $CO_2$- Parameter und endexspiratorischen $CO_2$- Messwerte als besondere Ausgestaltung, bzw. Untermenge der exspiratorischen $CO_2$-Parameter und exspiratorischen $CO_2$- Messwerte in der Beschreibung gemeinsam erläutert und nebeneinander mittels Kurzzeichen, Abkürzungen, Bezugsziffern und Formeln, beispielsweise in der Form ($CO_2$; et$CO_2$), verwendet.

Im messtechnischen Idealfall erfasst ein zum Beispiel in einer Atemmaske platzierter $CO_2$-Sensor während und gegen Ende der Ausatmung den $CO_2$-Gehalt von alveolarer Lungenluft. Bedingt durch die Physiologie steht deren $CO_2$-Partialdruck ($pCO_2$), insbesondere der endtidale $CO_2$-Partialdruck ($petCO_2$) am Ende der Ausatmung dabei nahezu im Gleichgewicht mit dem arteriellen $CO_2$-Partialdruck des arteriellen Bluts ($PaCO_2$) eines Patienten.

Im messtechnisch ungünstigsten Fall wird mittels des $CO_2$-Sensors lediglich ein mittlerer $CO_2$-Wert erfasst, der sich kaum zwischen Inspiration und Exspiration unterscheidet. Dieses kann z.B. durch den Messort in der Maske bedingt sein, an dem sich inspiratorische und exspiratorische Gase mischen. Auch in diesem Fall gibt es den physiologischen Zusammenhang zwischen der exspiratorisch, bzw. endexspiratorisch gemessenen $CO_2$-Konzentration ($CO_2$; et$CO_2$), dem $CO_2$-Partialdruck ($PCO_2$; $PetCO_2$) mit dem arteriellen $CO_2$-Partialdruck des arteriellen Bluts ($PaCO_2$). Allerdings ist der Messwert beeinflusst von Faktoren wie zum Beispiel dem Atmungsmuster (I:E-Verhältnis) und einer Leckage der Maske (Einflussfaktoren). Solange diese Einflussfaktoren sich im Zeitverlauf von Beatmung, bzw. Behandlung des Patienten nicht ändern, bleibt dieser Zusammenhang - gleichsam als eine Korrelation - erhalten. Dieser Zusammenhang gilt in vergleichbarer Weise auch dann, wenn die in Bezug genommenen Werte zu annähernd den selben Zeitpunkten während der Ausatmung gewonnen werden, ebenfalls unter der Randbedingung, dass sich diese Einflussfaktoren sich im Zeitverlauf von Beatmung, bzw. Behandlung des Patienten nicht ändern und die Zeitpunkte der Datenermittlung und Datengewinnung während der Ausatmung sich im Zeitverlauf von Beatmung, bzw. Behandlung des Patienten nicht ändern.

Die mittels einer Sensorik, zum Beispiel einer Sensorik in einer Atemmaske, messtechnisch erfasste exspiratorische $CO_2$-Konzentration (Messwert), bzw, endexspiratorische $CO_2$-Konzentration (Messwert) wird im Folgen-

den als $CO_2$mess, bzw. et$CO_2$mess bezeichnet. Diese hängt - wie dargestellt - individuell vom Patienten sowie der Beatmungssituation ab. Angestrebt wird eine Optimierung der $CO_2$-Messung unter Maskenbeatmung, bei welcher der erfasste Messwert ($CO_2$mess; et$CO_2$mess) möglichst wenig von den Einflussfaktoren abhängt, so dass die Korrelation mit $PaCO_2$ auch bei Änderungen der Einflussfaktoren erhalten bleibt. Dafür ist vorgesehen, dass zum einen von einem als akzeptabel bewerteten Messwert als Startwert ("Snapshot") ausgegangen wird und dass zum anderen zeitlich nachfolgende Messwerte in Bezug auf den Startwert normiert werden. Die Festlegung eines Startwerts, also die Auswahl eines Messwerts als akzeptabel, erfolgt durch medizinisch geschultes Personal, insbesondere einen Arzt.

Der im Folgenden als $CO_2$equal, bzw. et$CO_2$equal bezeichnete Trendparameter ergibt sich als einheitenloser Quotient (einheitenloser Trendparameter) aus einem jeweils aktuellen Messwert $CO_2$mess(k), bzw. et$CO_2$mess(k) und dem festgelegten Startwert, wobei der Startwert der zu einem Startzeitpunkt (k=0) aufgenommene Messwert $CO_2$mess(k=0), bzw. et$CO_2$mess(k=0) ist:

$$CO_2equal = CO_2mess(k) / CO_2mess(0)$$

$$bzw.\ etCO_2equal = etCO_2mess(k) / etCO_2mess(0)$$

Zum Zeitpunkt k=0 wird dieser Quotient vorzugsweise auf 1.0 normiert, alternativ kann die Normierung auch auf 100% erfolgen. So ergibt sich zum Zeitpunkt k=0 für den Trendparameter $CO_2$equal, bzw. et$CO_2$equal der Wert 1,0,bzw. 100%. Im weiteren Verlauf der Beatmung schwankt der Trendparameter dann um 1,0, bzw. um 100%. Die angestrebte Unabhängigkeit des resultierenden Trendparameters $CO_2$equal, bzw. et$CO_2$equal von eventuellen Änderungen der Einflussfaktoren ergibt sich auf diese Weise mittels der anfänglichen Ermittlung eines akzeptablen Startwerts und der späteren Quotientenbildung. Der akzeptable Startwert kann beispielsweise anhand einer Bewertung eines klinischen Anwenders definiert werden oder sich aus Tabellen mit Bewertungen und Zuordnungen des Quotienten zu bestimmten Patientenzuständen ergeben.

Der Trendparameter kann als Regelgröße (Istwert) einer Regelung verwendet werden. Als Sollwert (Führungsgröße) kann aufgrund der Normierung des Trendparameters der Wert 1,0 bzw. 100% verwendet werden. Mittels der Regelung wird angestrebt, den Trendparameter konstant zu halten. Mittels resultierender Stellgrößen beeinflusst die Regelung zum Beispiel eine Fördervorrichtung des $CO_2$-Austauschers (EC$CO_2$R) und/oder einen Ventilator des $CO_2$-Austauschers. Eine die Fördervorrichtung beeinflussende Stellgröße beeinflusst die durch den $CO_2$-Austauscher strömende Blutmenge (Blutflussrate). Eine den Ventilator beeinflussende Stellgröße beeinflusst den Gasaustausch im Blut innerhalb des $CO_2$-Austauschers. Ein Ausführungsbeispiel der Regelung ist im speziellen Beschreibungsteil erläutert. Ausgehend von dem Ansatz einer Regelung auf Basis eines Trendparameters hat sich herausgestellt, dass eine noch einfachere Form einer Regelung möglich ist, indem - kurz gefasst - mittels der Regelung versucht wird, eine als akzeptabel festgehaltene exspiratorische, bzw. endexspiratorische $CO_2$-Konzentration im Atemgas konstant zu halten oder zumindest im Wesentlichen konstant zu halten.

Damit wird die oben genannte Aufgabe erfindungsgemäß mittels eines Systems mit den Merkmalen des Anspruchs 1 gelöst.

Dazu ist bei einem solchen System zur Unterstützung des Blutgasaustauschs eines Patienten mittels Beatmung einerseits sowie mittels extrakorporalem Blutgasaustausch mittels eines $CO_2$-Austauschers andererseits vorgesehen,

- dass mittels einer Sensorik, insbesondere einer von dem System umfassten oder dem System zugeordneten Sensorik, ein Messwert ($CO_2$mess; et$CO_2$mess) bezüglich einer exspiratorischen $CO_2$-Konzentration (Kohlendioxidkonzentration) im Atemgas des Patienten erfassbar ist und beim Betrieb des Systems erfasst wird,
- dass mittels einer Bedienhandlung, insbesondere einer Bedienhandlung an einer Eingabevorrichtung eines von dem System umfassten Geräts, ein aktueller Messwert bezüglich der exspiratorischen, bzw. endexspiratorischen $CO_2$-Konzentration im Atemgas ($CO_2$mess(0); et$CO_2$mess(0)) als Startwert auswählbar ist und beim Betrieb des Systems durch einen Bediener des Systems ausgewählt wird
- und dass der Startwert als Basis für eine Regelung mittels eines Reglers fungiert,
- wobei der Regler auf einen $CO_2$-Austauscher wirkt, also zum Beispiel eine von dem Regler ausgegebene Stellgröße als Sollwert für den $CO_2$-Austauscher fungiert.

In einer vorzugsweisen Ausführungsform ist mittels der Sensorik als Messwert bezüglich der exspiratorischen, $CO_2$-Konzentration im Atemgas des Patienten ein Messwert bezüglich einer endexspiratorischen CO2-Konzentration (et$CO_2$) im Atemgas des Patienten erfassbar. Die Wahl der endexspiratorischen CO2-Konzentration (et$CO_2$), oftmals auch als endtidale Kohlendioxidkonzentration (et$CO_2$) bezeichnet, ermöglicht es, zu sich im Rhythmus der Atmung wiederholenden Zeitpunkten definiert zu annähernd gleichen Zuständen des Patienten Messwerte zu erfassen, welche dann als sowohl als sich ständig im Zeitverlauf der Therapie und Beatmung aktualisierende Eingangsgröße für den Regler dient, als auch zu Beginn der Therapie bei der Gewinnung des Startwertes Verwendung findet. Werden als exspiratorische $CO_2$-Konzentrationsmesswerte im Atemgas des Patienten endexspiratorischer CO2-Konzentrationmesswerte ($etCO_2$) verwendet, so ergibt sich eine Synchronisierung der Messwertabtastung für die Regelung in Anlehnung an die Beatmungssteuerung, so dass durch den Anwender im Betrieb Änderungen der Beatmungssteuerung, z.B. der Beatmungsfrequenz vorgenommen werden können, ohne dass die Messwertabtastung zusätzlich einer Anpassung bedarf, da der $CO_2$- Messwert unabhängig von der Einstellungsveränderung am Ende der Ausatmung gewonnen wird. Dies ergibt den Vorteil einer Robustheit bei der Messwerterfassung und damit mittelbar auch in der erfindungsgemäßen Regelung mit Wirkung auf den $CO_2$- Austauscher gegenüber Ausgestaltungen anderer Ausführungsformen. Zudem können in der Praxis Lösungen mit $CO_2$-Austauscher gestaltet werden, dessen Regelung mittels der vom Regler ausgegebenen Stellgröße als Sollwert für den $CO_2$-Austauscher erfindungsgemäß auch ohne Dateninformationen von einem Beatmungsgerät oder einer Beatmungssteuerung ermöglicht sein kann.

In anderen Ausführungsformen können zu beliebigen jedoch im Zeitablauf der Beatmung definierten Zeitpunkten während der Ausatmung Messwerte von der Sensorik erfasst werden. In solchen Ausführungsformen ist die zeitliche Lage der Messwerterfassung innerhalb der Exspirationsphase geeignet anhand von Ereignissen des Beatmungsablaufs oder Zeitpunkten zu definieren und mit Hilfe einer zur Beatmungssteuerung mit Beatmungsfrequenz (RR) und Inspirations- zu Exspirationsverhältnis (I:E- Ratio) koordinierten Zeitsteuerung dann angepasst an jeweilige Beatmungseinstellungen (RR, I:E) anzupassen.

Die zur Regelung erforderliche Datengewinnung exspiratorischer $CO_2$-Konzentrationen im Atemgas gegen - insbesondere auch exakt - am Ende ($etCO_2$) der Ausatmung stellt zudem den Vorteil bereit, dass die Strömungsverhältnisse am Messort (z.B. am Y- Stück) der Erfassung der $CO_2$-Konzentration im Atemgas zu diesen Zeitpunkten auch während eines längerfristigen Zeitverlaufs von der Beatmung, bzw. Behandlung des Patienten vergleichsweise stabil bleiben, sodass die messtechnisch erfassten - und zur Regelung des $CO_2$- Absorbers verwendeten - Veränderungen der endexspiratorischen $CO_2$-Konzentrationen im Zeitverlauf von Beatmung, bzw. Behandlung des Patienten im Wesentlichen nicht durch Strömungseffekte verursacht oder von diesen überlagert sind.

Die Funktion des Startwerts als Basis für die Regelung kann in zwei Formen erfolgen.

1. Zum einen kann in einer nicht beanspruchten Form der Startwert als Basis für eine Regelung fungieren indem eine Differenz aus dem Startwert und einem jeweils aktuellen Messwert dem auf den $CO_2$-Austauscher wirkenden Regler zuführbar ist und dem Regler beim Betrieb des Systems zugeführt wird.

2. Zum anderen fungiert erfindungsgemäß der Startwert als Basis für eine Regelung, indem eine Differenz aus einem mit dem Startwert gebildeten Trendparameter und einem Sollwert für den Trendparameter dem auf den $CO_2$-Austauscher wirkenden Regler zuführbar ist und dem Regler beim Betrieb des Systems zugeführt wird.

Bei einer Regelung auf Basis der Differenz aus dem mit dem Startwert gebildeten Trendparameter und dem Sollwert für den Trendparameter ist optional vorgesehen, dass der Trendparameter ($CO_2equal$; $etCO_2equal$) mit dem Startwert ($CO_2mess(0)$; $etCO_2mess(0)$) einerseits sowie einem jeweils aktuell ermittelten Messwert ($CO_2mess(k)$; $etCO_2mess(k)$) andererseits, nämlich einem Messwert bezüglich der exspiratorischen bzw. endexspiratorischen $CO_2$-Konzentration im Atemgas, ermittelbar ist - zum Beispiel in Form der erwähnten Quotientenbildung - und beim Betrieb des Systems ermittelt wird. Alternativ zur Quotientenbildung kann auch eine - beispielsweise gewichtete Differenzenbildung zur Bestimmung des Trendparameters ($CO_2equal$; $etCO_2equal$) gewählt werden. Eine Differenz aus dem Sollwert für den Trendparameter, zum Beispiel 1,0 bzw.100%, sowie einem jeweils aktuellen Wert des Trendparameters ist einem Regler zuführbar und wird dem Regler zugeführt, wobei der Regler auf den $CO_2$-Austauscher wirkt, also zum Beispiel eine von dem Regler ausgegebene Stellgröße als Sollwert für den $CO_2$-Austauscher fungiert.

Aufgrund der bei beiden Situationen (Regelung auf Basis eines als Startwert festgehaltenen Messwerts sowie eines aktuellen Messwerts bzw. Regelung auf Basis des Trendparameters) gleichen Regelstrecke, also dem Gasgehalts des Bluts des Patienten, wird davon ausgegangen, dass die Qualität beider Regelungsmöglichkeiten gleich oder zumindest im Wesentlichen gleich ist.

Mittels der Regelung wird ein Konstanthalten der ursprünglich als akzeptabel festgehaltenen exspiratorischen, bzw. endexspiratorischen $CO_2$-Konzentration im Atemgas (Startwert) oder ein Konstanthalten des Trendparameters angestrebt. Die Regelung wird damit insgesamt auch als Erhaltungsregelung bezeichnet. Bei einer Regelung in Bezug auf den Trendparameter, welcher durch die Bildung einerseits mit dem Startwert und andererseits mit einem jeweils aktuell erfassten Messwert auf den Startwert bezogen ist, wird mit dem Konstanthalten des Trendparameters erreicht, dass die in Form des Messwerts in den Trendparameter eingehende exspiratorische, bzw. endexspiratorische $CO_2$-Konzentration im Atemgas des Patienten zumindest in der Umgebung des Startwerts verbleibt. Der Startwert wird als Startwert ausgewählt, weil dieses von einem Bediener des Systems, üblicherweise einem Arzt, als akzeptabel bewertet wurde. Mittels der Regelung wird damit erreicht, dass die exspiratorische, bzw. endexspiratorische $CO_2$-Konzentration im Atemgas des Patienten zumindest in der Umgebung eines als medizinisch akzeptabel angesehenen Werts bleibt.

Die oben genannte Aufgabe wird ebenso durch die Verwendung eines Systems der hier und im Folgenden beschriebenen Art gelöst. Mittels des Systems und einer von dem System umfassten oder dem System zugeordneten Sensorik wird ein Messwert als Maß für eine exspiratorische, bzw. endexspiratorische $CO_2$-Konzentration im Atemgas des Patienten erfasst und optional angezeigt. Durch einen Bediener des Systems wird mittels einer Bedienhandlung ein Vorliegen einer akzeptablen $CO_2$-Konzentration im Atemgas bestätigt. Auf eine solche Bedienhandlung erfolgt im Rahmen eines Betriebs des Systems ein geregelter Betrieb des von dem System umfassten $CO_2$-Austauschers, mit dem Ziel einer Beibehaltung der als akzeptabel gekennzeichneten $CO_2$-Konzentration. Beim geregelten Betrieb des $CO_2$-Austauschers erfolgt eine Abfuhr von Kohlendioxid aus dem Blut des Patienten. Der geregelte Betrieb des $CO_2$-Austauschers kann in Form eines durch die Regelung bewirkten oder zumindest angestrebten Konstanthaltens der ursprünglich als akzeptabel ausgewählten akzeptablen $CO_2$-Konzentration oder eines Konstanthaltens eines mit der ursprünglich als akzeptabel ausgewählten $CO_2$-Konzentration gebildeten Trendparameters erfolgen.

Das System wird automatisch, also ohne einen besonderen Eingriff des Benutzers des Systems, betrieben. Der automatische Betrieb erfolgt unter Kontrolle einer Steuerungseinheit. Diese umfasst eine Verarbeitungseinheit in Form von oder nach Art eines Mikroprozessors sowie einen Speicher. In den Speicher ist ein von der Verarbeitungseinheit ausführbares Steuerungsprogramm geladen oder ladbar, das beim Betrieb des Systems durch die Verarbeitungseinheit ausgeführt wird.

Die Erfindung ist bevorzugt in Software implementiert. Die Erfindung ist damit einerseits auch ein Computerprogramm mit durch einen Computer ausführbaren Programmcodeanweisungen und andererseits ein Speichermedium mit einem derartigen Computerprogramm, also ein Computerprogrammprodukt mit Programmcodemitteln, sowie schließlich auch eine Steuerungseinheit oder ein Medizingerät, in deren bzw. dessen Speicher als Mittel zum automatischen Betrieb des Systems ein solches Computerprogramm geladen oder ladbar ist.

Ein Vorteil der Erfindung besteht darin, dass ein objektiv vergleichbares und reproduzierbares Kontrollverfahren für den $CO_2$-Partialdruck des Bluts ($PaCO_2$), ausgehend von einem ärztlich festgestellten stabilen Zustand - und dem dazu als Startwert festgehaltenen Messwert bezüglich der exspiratorischen bzw. endexspiratorischen $CO_2$-Konzentration im Atemgas - angegeben wird. Der hier vorgeschlagene Ansatz stellt auch bei unzureichender $etCO_2$-Messung aufgrund von Maskenleckagen noch einen optimalen oder mindestens akzeptablen $CO_2$-Partialdruck des Bluts sicher.

Vorteilhafte Ausgestaltungen der Erfindung sind Gegenstand der Unteransprüche.

Dabei verwendete Rückbeziehungen weisen auf die weitere Ausbildung des Gegenstandes des Hauptanspruches durch die Merkmale des jeweiligen Unteranspruches hin und sind nicht als ein Verzicht auf die Erzielung eines selbständigen, gegenständlichen Schutzes für die Merkmalskombinationen der rückbezogenen Unteransprüche zu verstehen. Des Weiteren ist im Hinblick auf eine Auslegung der Ansprüche sowie der Beschreibung bei einer näheren Konkretisierung eines Merkmals in einem nachgeordneten Anspruch davon auszugehen, dass eine derartige Beschränkung in den jeweils vorangehenden Ansprüchen sowie einer allgemeineren Ausführungsform des gegenständlichen Systems nicht vorhanden ist. Jede Bezugnahme in der Beschreibung auf Aspekte nachgeordneter Ansprüche ist demnach auch ohne speziellen Hinweis ausdrücklich als Beschreibung optionaler Merkmale zu lesen. Schließlich ist darauf hinzuweisen, dass das hier vorgeschlagene System auch weitergebildet sein kann, indem das System Mittel umfasst, die zur Ausführung eines oder mehrerer beim Betrieb des System ablaufender Verfahrensschritte bestimmt und/oder eingerichtet sind. Insoweit gelten Merkmale und Details, die im Zusammenhang mit dem vorgeschlagenen System zur Unterstützung des Blutgasaustauschs mittels Beatmung sowie extrakorporalem Blutgasaustausch und eventueller Ausgestaltungen beschrieben sind, selbstverständlich auch im Zusammenhang mit und im Hinblick auf beim Betrieb des Systems durch das System ausgeführte Verfahrensschritte und jeweils umgekehrt, so dass bezüglich der Offenbarung zu den einzelnen Aspekten der Erfindung stets wechselseitig Bezug genommen wird bzw. werden kann.

Erfindungsgemäß ist der Trendparameter ($etCO_2equal$) in Form einer Normierung des jeweils aktuell ermittelten Messwerts ($etCO_2mess(k)$) in Relation zu dem Startwert ($etCO_2mess(0)$) ermittelbar bzw. wird mittels einer solchen Normierung als einheitenloser Trendparameter ermittelt, nämlich durch Quotientenbildung:

$$etCO_2equal = etCO_2mess(k) \ / \ etCO_2mess(0).$$

Bei einer weiteren Ausführungsform des Systems ist der jeweils aktuelle Messwert bezüglich der exspiratorischen, bzw. endexspiratorischen $CO_2$-Konzentration im Atemgas an einer Anzeigeeinheit ausgebbar und wird beim Betrieb des Systems an der Anzeigeeinheit ausgegeben.

Mittels der Bedienhandlung ist der angezeigte Messwert als Startwert auswählbar und wird beim Betrieb des Systems als Startwert ausgewählt. Ohne eine solche Anzeigeeinheit - also bei der bisher beschriebenen Form des Systems - nimmt der Bediener des Systems, also üblicherweise ein Arzt, die den Startwert auswählende Bedienhandlung aufgrund einer Beobachtung des Patienten vor. Die Beobachtung zielt dabei darauf ab, ein stabiles Atemverhalten des Patienten (ruhiger bestimmter Atemantrieb) zu erkennen. Mit einer solchen Anzeigeeinheit kann der Bediener des Systems einerseits das beobachtete Atemverhalten des Patienten sowie andererseits den jeweils angezeigten Messwert berücksichtigen und danach den Zeitpunkt für seine Bedienhandlung, welche das Festhalten des Startwerts und in Folge den Beginn der Erhaltungsregelung auslöst, bestimmen.

Bei einer bevorzugten Ausführungsform des Systems ist mittels einer Sensorik eine Spontanatemfrequenz des Patienten überwachbar und wird beim Betrieb des Systems überwacht, wobei im Falle einer Überschreitung eines vorgegebenen oder vorgebbaren Schwellwerts ein Signalelement ansteuerbar ist und beim Betrieb des Systems im Falle einer solchen Schwellwertüberschreitung angesteuert wird.

Bei einer vorteilhaften Ausführungsform des Systems ist mittels einer Bedienhandlung am System ein Entwöhnungsmodus aktivierbar und wird beim Betrieb des Systems im Falle einer entsprechenden Entscheidung eines Bedieners des Systems durch eine Bedienhandlung des Bedieners aktiviert, wobei im Entwöhnungsmodus eine $CO_2$-Abbau-Sollrate automatisch und gesteuert reduzierbar ist und im Rahmen des Betriebs des Systems reduziert wird.

Bei einer speziellen Ausführungsform der vorgenannten vorteilhaften Ausführungsform des Systems ist beim oder im Zusammenhang mit dem Beginn der Reduktion der $CO_2$-Abbau-Sollrate die Erhaltungsregelung deaktivierbar, zum Beispiel indem ein Einfluss des Reglers auf den $CO_2$-Austauscher deaktivierbar ist. Der Trendparameter wird aber trotzdem weiter ermittelt. Dieser ist während der Reduktion der $CO_2$-Abbau-Sollrate in Bezug auf einen vorgegebenen oder vorgebbaren Toleranzbereich überwachbar. Bei einer Verletzung des Toleranzbereichs ist einerseits die Erhaltungsregelung wieder aktivierbar, zum Beispiel indem der Einfluss des Reglers auf den $CO_2$-Austauscher wieder aktiviert wird, und andererseits die bisherige Reduktion der $CO_2$-Abbau-Sollrate deaktivierbar. Bei einem entsprechenden Betrieb des Systems wird beim oder im Zusammenhang mit dem Beginn der Reduktion der $CO_2$-Abbau-Sollrate die Erhaltungs- regelung deaktiviert. Der weiterhin ermittelte Trendparameter wird während der Reduktion der $CO_2$-Abbau-Sollrate in Bezug auf einen vorgegebenen oder vorgebbaren Toleranzbereich überwacht. Bei einer Verletzung des Toleranzbereichs wird einerseits die Erhaltungsregelung wieder aktiviert und andererseits die bisherige Reduktion der $CO_2$-Abbau-Sollrate deaktiviert.

Mit dieser Ausführungsform des Systems ist eine automatische und automatisch überwachte Entwöhnung des Patienten von der Wirkung des $CO_2$-Austauschers möglich. Die Entwöhnung besteht in Form der Reduktion der $CO_2$-Abbau-Sollrate. Durch die auch während der Entwöhnung erfolgende Ermittlung des Trendparameters kann der Verlauf der Entwöhnung überwacht werden. Wenn der Trendparameter einen Toleranzbereich verlässt, wird die Reduktion der $CO_2$-Abbau-Sollrate gestoppt und die Erhaltungsregelung wird aktiviert. Wenn sich im Rahmen der Erhaltungsregelung wieder ein zulässiger und/oder stabiler Trendparameter einstellt, kann die Erhaltungsregelung wieder gestoppt und die Reduktion der $CO_2$-Abbau-Sollrate erneut beginnen. Wenn sich hier erneut eine Verletzung des Toleranzbereichs ergibt, wird die Erhaltungsregelung wieder aktiviert und so weiter. Diese automatische Entwöhnung des Patienten kann so lange durchgeführt werden, bis bezüglich der $CO_2$-Abbau-Sollrate ein Zielwert erreicht ist.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen.

Das Ausführungsbeispiel ist nicht als Einschränkung der Erfindung zu verstehen. Vielmehr sind im Rahmen der vorliegenden Offenbarung Abänderungen und Modifikationen möglich, insbesondere solche Varianten und Kombinationen, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen für den Fachmann im Hinblick auf die Lösung der Aufgabe entnehmbar sind und durch kombinierbare Merkmale zu einem neuen Gegenstand führen.

Es zeigen:

Figur 1    ein System zur Beatmung eines Patienten, welches zumindest ein als Beatmungsgerät fungierendes Medizingerät umfasst,

Figur 2    ein in dem System gemäß Figur 1 als Steuerungseinheit fungierendes Gerät,

Figur 3    ein Regelkreis zur Regelung des extrakorporalen Blutaustauschs eines Patienten,

Figur 4    einen Verlauf einer Entwöhnung eines Patienten von einem extrakorporalen Blutaustausch und

Figur 5    ein Flussdiagram zur Veranschaulichung eines bei der Entwöhnung gemäß Figur 4 ablaufenden Verfahrens.

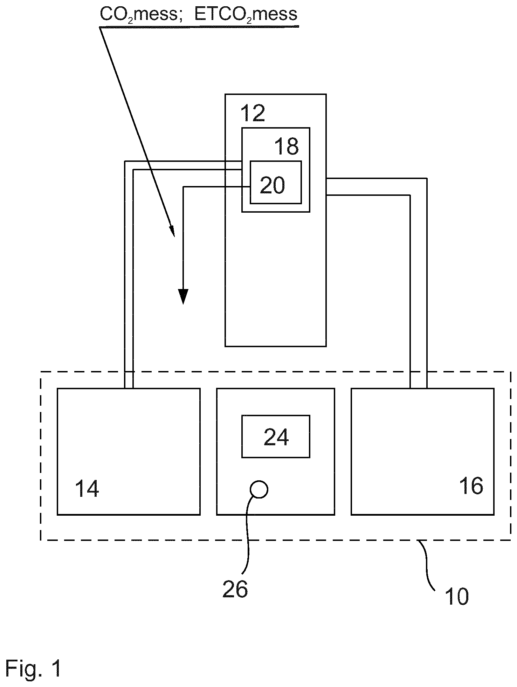

Die Darstellung in Figur 1 zeigt - schematisch stark vereinfacht - ein System 10 zur Unterstützung eines Gasaustauschs bei einem Patienten 12. Das System 10 umfasst zumindest zwei Medizingeräte 14, 16, nämlich ein erstes Medizingerät 14 in Form einer Beatmungsvorrichtung zur maschinellen Beatmung einer Lunge des Patienten 12 und ein zweites Medizingerät 16 zum extrakorporalen Blutgasaustausch. Mittels des zweiten Medizingeräts 16 erfolgt zumindest eine Abfuhr von Kohlendioxid ($CO_2$) aus dem Blut des Patienten 12 und ggf. eine Anreicherung des Bluts mit Sauerstoff ($O_2$). Im Folgenden wird das als Beatmungsvorrichtung fungierende erste Medizingerät 14 kurz - aber ohne Verzicht auf eine weitergehende Allgemeingültigkeit - als Beatmungsgerät 14 bezeichnet. Entsprechend wird das zweite, zumindest zur Abfuhr von Kohlendioxid aus dem Blut des Patienten 12 bestimmte Medizingerät 16 kurz - aber ebenfalls ohne Verzicht auf eine weitergehende Allgemeingültigkeit - als $CO_2$-Austauscher 16 bezeichnet. Geräte 14, 16 der vorgenannten Art sind an sich bekannt. Ein $CO_2$-Austauscher 16 wird in der Fachliteratur mitunter auch kurz als $ECCO_2R$ bezeichnet.

Das Beatmungsgerät 14 ist in an sich bekannter Art und Weise nicht-invasiv an die Lunge des Patienten 12 angeschlossen, zum Beispiel mittels einer Atemmaske 18. Der $CO_2$-Austauscher 16 ist in ebenfalls an sich bekannter Art und Weise an den Blutkreislauf des Patienten 12 angeschlossen.

Mittels einer zumindest einen $CO_2$-Sensor umfassenden Sensorik 20, zum Beispiel einer in grundsätzlich an sich bekannter Art und Weise in der Atemmaske 18 befindlichen Sensorik 20, ist beim Betrieb des Beatmungsgeräts 14 und insgesamt beim Betrieb des Systems 10 ein Messwert bezüglich einer exspiratorischen, bzw. endexspiratorischen $CO_2$-Konzentration im Atemgas des Patienten erfassbar. Dieser Messwert wird im Folgenden mitunter kurz mit $etCO_2mess$ bezeichnet.

Innerhalb des Systems 10 fungiert eines der zumindest zwei Medizingeräte 14, 16, zum Beispiel das Beatmungsgerät 14, als übergeordnetes Gerät oder das System 10 umfasst ein dediziertes übergeordnetes Gerät oder ein als übergeordnetes Gerät und als Steuerungseinheit fungierendes weiteres Medizingerät 22. Die nachfolgende Beschreibung wird anhand eines Systems 10 mit einem solchen weiteren Medizingerät 22 fortgesetzt und dieses wird als Steuerungseinheit 22 bezeichnet. Grundsätzlich kommt genauso in Betracht, dass das System 10 kein solches weiteres Medizingerät 22 umfasst und dass stattdessen das Beatmungsgerät 14 oder auch der $CO_2$-Austauscher 16 als Steuerungseinheit fungiert. Dies ist im Folgenden bei jeder Erwähnung eines übergeordneten Geräts oder einer Steuerungseinheit stets mitzulesen. Bei einer separaten Steuerungseinheit 22 ist diese mit den sonstigen Geräten 14, 16 des Systems 10 zumindest zum Austausch von Daten, insbesondere in Form von Messwerten und/oder Steuersignalen, in grundsätzlich an sich bekannter Art und Weise kommunikativ verbunden. Im Falle eines Systems 10 ohne eine solche separate Steuerungseinheit ist das als Steuerungseinheit fungierende Gerät 14, 16 mit den sonstigen Geräten 14, 16 des Systems in der vorstehend skizzierten Art und Weise verbunden.

Das als Steuerungseinheit 22 fungierende Gerät umfasst bei der gezeigten Ausführungsform eine Anzeigeeinheit 24 sowie eine Eingabevorrichtung 26 oder ist in grundsätzlich an sich bekannter Art und Weise mit einem Gerät oder Geräten mit einer Anzeigeeinheit 24 und/oder eine Eingabevorrichtung 26 verbunden. Der mittels der Sensorik 20 aufnehmbare und beim Betrieb des Systems 10 regelmäßig aufgenommene Messwert ($etCO_2mess$) wird an das System 10 ausgegeben und innerhalb des Systems 10 verarbeitet. Eine Anzeigeeinheit 24 ist optional. Die Ausgabe des Messwerts erfolgt unmittelbar oder mittelbar an das als Steuerungseinheit 22 fungierende Gerät und die Verarbeitung des Messwerts umfasst im Falle einer vorhandenen Anzeigeeinheit 24 zumindest eine Darstellung des Messwerts mittels der Anzeigeeinheit 24. Die Anzeige des Messwerts mittels der Anzeigeeinheit 24 ermöglicht einem Bediener des Systems 10, üblicherweise einem Arzt, die Überwachung des Messwerts.

Ohne eine solche Anzeige des Messwerts beurteilt der Bediener den Patienten 12 selbst, also zum Beispiel dessen Atemverhalten. Wenn der Bediener ein zufriedenstellendes Atemverhalten des Patienten 12 und/oder einen mittels der Anzeigeeinheit 24 ausgegebenen Messwert in einem akzeptablen Bereich erkennt oder einen über einen ausreichend langen Zeitraum stabil in einem akzeptablen Bereich verbleibenden Messwert erkennt, nimmt dieser eine Bedienhandlung an dem System 10 vor. Mittels der Bedienhandlung wird ausgedrückt, dass ein zufriedenstellendes Atemverhalten und/oder ein akzeptabler angezeigter Messwert vorliegt. Bei einem zufriedenstellenden Atemverhalten als alleinigem Kriterium für die Vornahme der Bedienhandlung kann davon ausgegangen werden, dass der - unabhängig von einer eventuellen Anzeige mittels der Anzeigeeinheit 24 - mittels der Sensorik 20 aufgenommene Messwert ($etCO_2mess$) ebenfalls ein akzeptabler Messwert oder ein Messwert in einem akzeptablen Bereich ist. Mittels der Bedienhandlung, zum Beispiel einer Bedienhandlung in Form einer Betätigung der Eingabevorrichtung 26, ist der aufgenommene, aktuelle und optional angezeigte Messwert als Startwert für einen nach dem hier vorgeschlagenen Ansatz erfolgenden reproduzierbaren extrakorporalen $CO_2$-Austausch mittels des $CO_2$-Austauschers 16 auswählbar.

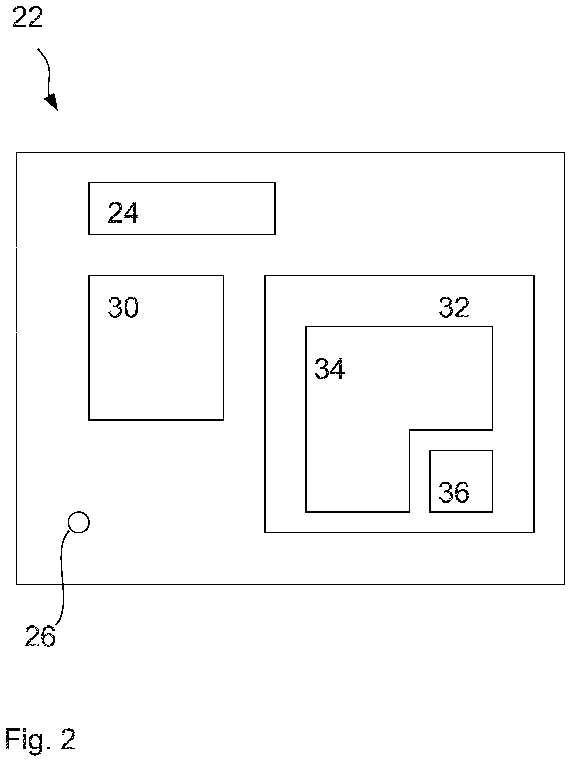

Die Darstellung in Figur 2 zeigt - ebenfalls schematisch stark vereinfacht - das in dem System 10 (Fig. 1) als Steuerungseinheit 22 fungierende Gerät, also zum Beispiel das weitere Medizingerät 22, wobei es sich bei dem weiteren Medizingerät 22 zum Beispiel um ein Medizingerät in Form eines dem Beatmungsgerät 14 und dem $CO_2$-Austauscher 16 zugeordneten oder hierarchisch übergeordneten Therapiegeräts oder dergleichen handelt. Die Steuerungseinheit 22 umfasst die (optionale) Anzeigeeinheit 24 und die Eingabevorrichtung 26. Dabei kann es sich jeweils um Elemente einer in grundsätzlich an sich bekannter Art und Weise mittels eines Monitors der Steuerungseinheit 22 dargestellten Benutzeroberfläche handeln. Die Steuerungseinheit 22 umfasst des Weiteren eine Verarbeitungseinheit 30 in Form von oder nach Art eines Mikroprozessors sowie einen Speicher 32, in den ein Steuerungsprogramm 34 geladen ist, das beim Betrieb der Steuerungseinheit 22 mittels der Verarbeitungseinheit 30 ausgeführt wird. Unter Kontrolle des Steuerungsprogramms 34 erfolgen zum Beispiel auch die Darstellung der Benutzeroberfläche und die Auswertung von Bedienhandlungen, die sich auf die Benutzeroberfläche beziehen, wenn die Steuerungseinheit 22 eine solche Benutzeroberfläche nutzt. Jedenfalls erfolgen unter Kontrolle des Steuerungsprogramms 34 die Ansteuerung der Anzeigeeinheit 24 und damit die Anzeige des Messwerts und die Auswertung der Eingabevorrichtung 26 zum Erkennen eventueller Bedienhandlungen.

Wenn durch eine solche Bedienhandlung - entweder aufgrund einer Beobachtung des Patienten 12 oder aufgrund einer Beobachtung der Anzeigeeinheit 24 - ein mittels der Sensorik 20 aufgenommener Messwert als akzeptabler Messwert ausgewählt wurde, wird dieser als Startwert zum Beispiel in einer Startwertspeicherstelle 36 im Speicher 32 festgehalten und dafür in die Startwertspeicherstelle 36 geladen.

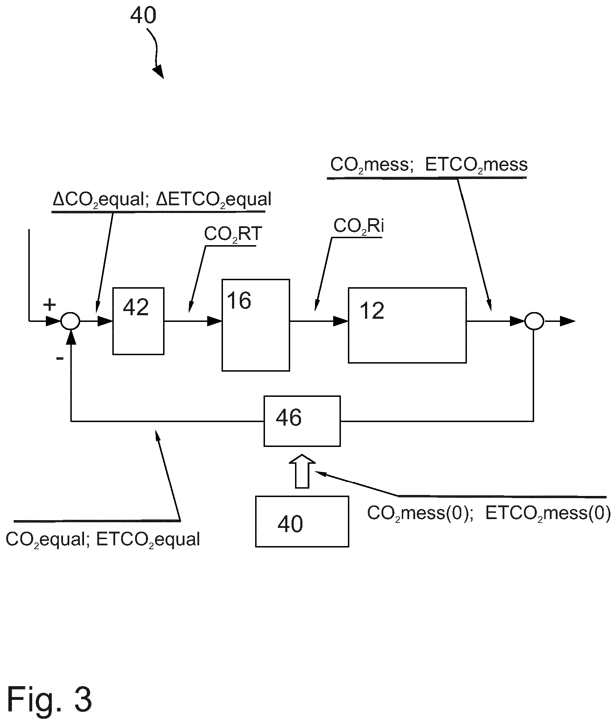

Die Darstellung in Figur 3 zeigt einen Regelkreis 40 zur Implementation der im allgemeinen Beschreibungsteil erläuterten Regelung zum Konstanthalten eines Trendparameters, dem ein jeweils aktuell ermittelter Messwert zugrunde liegt (Erhaltungsregelung). Für eine Regelung zum Konstanthalten des Startwerts gilt das Folgende entsprechend und eine Regelung, welche anstelle eines Konstanthaltens des Trendparameters ein Konstanthalten des Startwerts zum Ziel hat, ist entsprechend stets mitzulesen.

Der Regelkreis 40 umfasst in grundsätzlich an sich bekannter Art und Weise einen Regler 42, zum Beispiel einen P-Regler, einen PI-Regler oder einen PID-Regler, bevorzugt einen PI-Regler. Der Regler 42 wirkt auf den $CO_2$-Austauscher 16 und die Regelstrecke sind der $CO_2$-Austauscher 16 und der Patient 12. Der $CO_2$-Austauscher 16 wirkt mit einer $CO_2$-Abbaurate ($CO_2$ removal rate; $CO_2Ri$) auf den Patienten 12. Der $CO_2$-Austauscher 16 wird in grundsätzlich an sich bekannter Art und Weise mit einer als Sollwert für den $CO_2$-Austausch fungierenden $CO_2$-Abbau-Sollrate ($CO_2$ removal target; $CO_2Rt$) angesteuert. Der $CO_2$-Austauscher 16 wird innerhalb des Regelkreises 40 vom Regler 42 angesteuert. Der Regler 42 gibt also als Stellgröße die $CO_2$-Abbau-Sollrate vor.

Der im Regelkreis 40, nämlich am Patienten 12, ermittelte Messwert ist der mittels einer grundsätzlich an sich bekannten Sensorik 20 (Fig. 1) im vom Patienten 12 ausgeatmeten Atemgas ermittelte Messwert bezüglich der exspiratorischen, bzw. endexspiratorischen $CO_2$-Konzentration ($etCO_2mess$). Im Rückführzweig des Regelkreises 40 wird der Messwert mit einem vor Aktivierung der Regelung ermittelten und zum Beispiel aus der Startwertspeicherstelle 36 abgerufenen Startwert 44 ($etCO_2mess(0)$) zum Erhalt eines Trendparameters ($etCO_2equal$) normiert. Dies erfolgt mittels eines Normierers 46. Die Normierung mittels des Normierers 46 umfasst die im allgemeinen Beschreibungsteil erläuterte Quotientenbildung. Dabei wird mit dem jeweils aktuellen Messwert bezüglich der exspiratorischen, bzw. endexspiratorischen $CO_2$-Konzentration ($etCO_2mess(k)$) und dem Startwert 44 der Trendparameter ($etCO_2equal$) ermittelt. Als Führungsgröße / Sollwert fungiert zum Beispiel der Wert 1,0 oder der Wert 100%.

Mit der Führungsgröße und dem im Rückführzweig zurückgeführten Trendparameter ergibt sich in grundsätzlich an sich bekannter Art und Weise durch Subtraktion die Regelabweichung ($\Delta etCO_2equal$). Wenn der aufgrund der jeweils erfassten exspiratorischen, bzw. endexspiratorischen $CO_2$-Konzentration ($etCO_2mess(k)$) ermittelte Trendparameter vom Sollwert abweicht, greift die Regelung ein, indem der Regler 42 die $CO_2$-Abbau-Sollrate verändert. Der Eingriff des Reglers 42 erfolgt dabei, indem bei einer Erhöhung des Trendparameters der Sollwert für die $CO_2$-Abbaurate ($CO_2$-Abbau-Sollrate) erhöht wird. Dadurch sinkt der $CO_2$-Gehalt im Blut. Dies führt zu einer Verringerung des Atemantriebs des Patienten 12. Eine Erhöhung des Trendparameters ist (wegen $etCO_2equal = etCO_2mess(k) / etCO_2mess(0)$) gleichbedeutend mit einer gegenüber dem Startwert ($etCO_2mess(0)$) erhöhten exspiratorischen, bzw. endexspiratorischen $CO_2$-Konzentration ($etCO_2mess(k)$) im Atemgas des Patienten 12.

Eine optionale Verbesserung bezüglich der Erfassung eines Maßes für die exspiratorische, bzw. endexspiratorische $CO_2$-Konzentration im Atemgas des Patienten 12 kann zum Beispiel darin bestehen, dass anhand einer Information über die Einatembemühungen, welche beispielweise durch die Spontanatemfrequenz ($f_{spontan}$) und/ oder das I:E-Verhältnis (I:E) indiziert sein kann, ein Schätzwert für $etCO_2mess$ unter idealen messtechnischen Bedingungen, also gewissermaßen ein idealer Messwert, ermittelt wird. Dieser ideale Messwert wird im Folgenden als $etCO_2ideal$ bezeichnet. Die Information bezüglich der Spontanatemfrequenz ($f_{spontan}$) und des I:E-Verhältnisses (I:E) kann entweder von einer zusätzlichen Sensorik oder einem in die Sensorik 20 (Fig. 1) integrierten Flowsensor stammen oder direkt von der Atemphasenerkennung des $CO_2$-Sensors. Unter der Annahme, dass die Beatmung des Patienten 12 mit vernachlässigbar geringer inspiratorischer $CO_2$-Konzentration erfolgt, erhält man unter der Annahme konstanter inspiratorischer und exspiratorischer $CO_2$-Konzentrationen folgenden Schätzwert für $etCO_2ideal$:

$$etCO_2ideal = CO_2(exsp) + CO_2(insp)*(I:E)$$

Im allgemeinen Fall zeitlich variabler $CO_2$-Konzentrationen $CO_2(t)$ kann folgende Formel verwendet werden, bei welcher die gesamte während eines Atemhubs (T) gemessene $CO_2$-Konzentration der Exspirationsphase (Te) zugeschlagen wird:

$$etCO_2ideal = \frac{1}{Te}\int_0^T CO_2(t)dt$$

Hierbei ist Te die Dauer der Exspiration und T die Dauer des Atemhubs, also T = Ti + Te und T = 1/f.

Im Grenzfall einer verschwindenden inspiratorischen $CO_2$-Konzentration ergibt sich daraus erwartungsgemäß:

$$etCO_2 ideal = \frac{1}{Te} \int_0^{Te} CO_2(t)dt = CO_2(exsp)$$

Im Grenzfall inspiratorisch wie exspiratorisch gleicher Konzentrationen (stark verschliffene Messwerte) ergibt sich:

$$etCO_2 ideal = \frac{1}{Te} \int_0^{T} CO_2 \, dt = \frac{T}{Te} CO_2 = (1 + I{:}E) \, CO_2$$

In jedem Fall wird davon ausgegangen, dass die nichtverschwindende $CO_2$-Konzentration während der Inspiration ein messtechnisches Artefakt darstellt, weshalb eine Korrektur von $etCO_2$mess zu $etCO_2$ideal günstig ist.

Die Berechnung von $etCO_2$equal erfolgt - analog zu dem zuvor Beschriebenen - durch Normierung auf den als medizinisch akzeptabel eingestuften Startwert von $etCO_2$ideal:

$$etCO_2 equal \, (k) = \frac{etCO_2 ideal(k)}{etCO_2 ideal(k = 0)}$$

Der Zeitpunkt k=0 ist - unabhängig von der Verwendung von $etCO_2$mess oder $etCO_2$ideal - der "Initialzeitpunkt", zu dem der Patient 12 durch den Arzt als "o.k." bewertet wird. Kompliziertere Formen der Berechnung von $etCO_2$equal unter Einbeziehung der Spontanatemfrequenz als Gewichtungsfaktor oder zur Fallunterscheidung sind ebenfalls möglich und sinnvoll. Der Regler 42, die Verarbeitung des Messwerts, die Rückführung im Regelkreis 40 und die Ermittlung der Regelabweichung am Eingang des Reglers 42 sind bevorzugt in Software implementiert. Insoweit zeigen die entsprechenden Details der Darstellung in Figur 3 einen Teil der Funktionalität des Steuerungsprogramms 34 der Steuerungseinheit 22. Optional kann mittels einer entsprechenden, grundsätzlich an sich bekannten Sensorik (nicht gezeigt) die Spontanatemfrequenz ($f_{spontan}$) des Patienten 12 überwacht werden. Eine signifikante Änderung, also eine einen vorgegebenen oder vorgebbaren Schwellwert (zum Beispiel einer Überschreitung einer Spontanatemfrequenz von 30/min) überschreitende Änderung der Spontanatemfrequenz während der zuvor beschriebenen Erhaltungsregelung, kann auf eine Verschlechterung der Qualität der Messung der exspiratorischen, bzw. endexspiratorischen $CO_2$-Konzentration (et-$CO_2$mess(k)) hindeuten. Der jeweilige Messwert geht unmittelbar in die Ermittlung des Trendparameters ein und die Qualität des Messwerts bestimmt damit die Qualität der Erhaltungsregelung. Daher ist optional vorgesehen, dass im Fall einer sensorischen Überwachung der Spontanatemfrequenz bei einer einen vorgegebenen oder vorgebbaren Schwellwert überschreitenden Änderung der Spontanatemfrequenz automatisch ein Alarm ausgelöst wird, zum Beispiel mittels eines optischen und/oder akustischen Signalelements. Aufgrund eines solchen Alarms kann das Bedienpersonal zum Beispiel den Sitz einer Beatmungsmaske oder dergleichen prüfen und gegebenenfalls korrigieren.

Bei einer solchen optionalen Ausführungsform ist die Auswertung eines die Spontanatemfrequenz kodierenden Messwerts und die Überwachung des Messwerts in Bezug auf den Schwellwert bevorzugt ebenfalls als Teil der Funktionalität des Steuerungsprogramms 34 der Steuerungseinheit 22 implementiert. Für sich genommen sind der Vergleich eines Messwerts mit einem Schwellwert und die Ansteuerung eines Signalelements im Falle einer Schwellwertüberschreitung trivial und demgemäß sind entsprechende Funktionselemente oder Programmcodeanweisungen hier nicht gezeigt.

Zusätzlich oder alternativ kann optional eine automatisch unterstützte "Entwöhnung" des Patienten 12 von der Unterstützung durch den $CO_2$-Austauscher 16 vorgesehen sein. Der Beginn einer solchen Entwöhnung wird vom Bediener des Systems 10 (Fig. 1) aktiviert, zum Beispiel in Form einer Bedienhandlung, zum Beispiel einer Betätigung einer Eingabevorrichtung 26, insbesondere einer weiteren Eingabevorrichtung (Taster, Schalter, Element einer Benutzeroberfläche oder dergleichen). Dann schaltet das System 10 in einen Entwöhnungsmodus. Im Entwöhnungsmodus ist die Erhaltungsregelung (Fig. 3) zumindest zunächst deaktiviert. Der Entwöhnungsmodus zeichnet sich dadurch aus, dass die Sollrate für den $CO_2$-Abbau ($CO_2$-Abbau-Sollrate; $CO_2$Rt) um einen vorgegebenen oder vorgebbaren Wert zurückgefahren wird, zum Beispiel um 3ml/min pro Stunde. Im Entwöhnungsmodus wird weiterhin dauerhaft der Trendparameter $etCO_2$equal aktuell ermittelt und überwacht. Wenn der jeweils aktuelle Wert des Trendparameters einen vorgegebenen oder vorgebbaren Toleranzbereich, zum Beispiel 100% $\pm$ 10%, verlässt, wird die Erhaltungsregelung wieder aktiviert (und dabei die $CO_2$-Abbau-Sollrate ggf. wieder erhöht).

Die aufgrund einer Verletzung des Toleranzbereichs wieder aktivierte Erhaltungsregelung bleibt solange aktiv, bis der weiterhin jeweils aktuell ermittelte Trendparameter $etCO_2$equal einmal den Sollwert, also zum Beispiel 100%, erreicht hat, oder bis jeweils aktuell ermittelte Trendparameter $etCO_2$equal während einer vorgegebenen oder vorgebbaren Zeitspanne innerhalb des Toleranzbereichs oder eines im Vergleich zum Toleranzbereich "schmaleren" Erhaltungsregelungstoleranzbands, zum Beispiel 100% ± 2%, 100% ± 3%, 100% ± 5% usw., bleiben. Dann wird die Erhaltungsregelung wieder automatisch deaktiviert und es beginnt erneut das Zurückfahren der Sollrate für den $CO_2$-Abbau ($CO_2$Rt) um den oben erwähnten vorgegebenen oder vorgebbaren Wert und ausgehend von dem beim Deaktivieren der Erhaltungsregelung gültigen Wert für die $CO_2$-Abbau-Sollrate.

Beim Betrieb des Systems 10 kann im Entwöhnungsmodus ein mehrfaches Umschalten zwischen dem Zurückfahren der Sollrate für den $CO_2$-Abbau ($CO_2$Rt) und dem automatischen Wiederaktivieren der Erhaltungsregelung stattfinden.

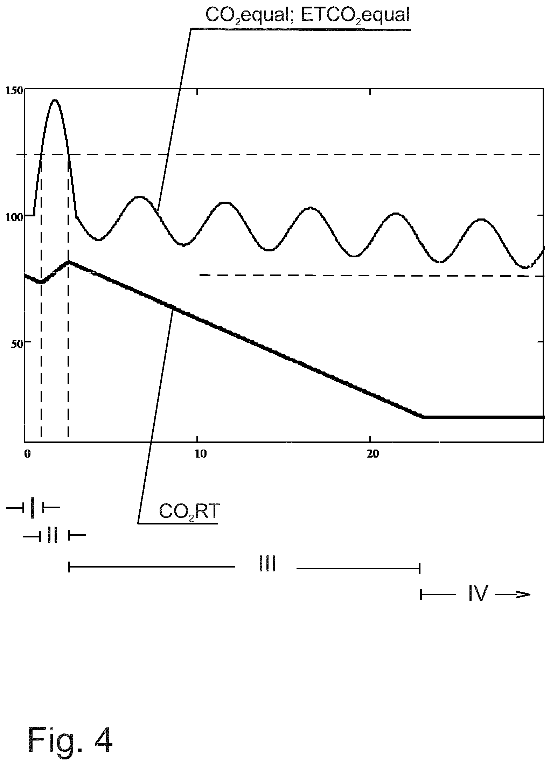

Die Darstellung in Figur 4 zeigt über der in Stunden aufgetragenen Zeit t einen möglichen Verlauf der Entwöhnung, nämlich im oberen Bereich einen Verlauf des Trendparameters (etCO2equal) und im unteren Bereich einen Verlauf der Sollrate für den $CO_2$-Abbau ($CO_2$Rt). Der Wertebereich des Verlaufs des Trendparameters ist auf der Ordinate in Prozentwerten angegeben (50%, 100%, 150%). Der Wertebereich des Verlaufs der Sollrate für den $CO_2$-Abbau ($CO_2$Rt) ist auf der Ordinate nicht zusätzlich angegeben. Im gezeigten Beispiel startet der Graph der Sollrate für den $CO_2$-Abbau bei 80 ml/min und endet bei 20 ml/min.

Gemäß der in Figur 4 exemplarisch dargestellten Situation wird zum Zeitpunkt t=0 der Entwöhnungsmodus aktiviert. Entsprechend fällt zunächst (Zeitabschnitt I) die Sollrate für den $CO_2$-Abbau. Der weiterhin ermittelte Trendparameter etCO2equal steigt allerdings an und steigt so sehr an, dass er eine Obergrenze eines mit zwei horizontalen gestrichelten Linien gezeigten und hier exemplarisch bei 100% ± 25% eingezeichneten Toleranzbereichs verletzt. Die Weite des Toleranzbereichs ist vorgegeben oder vorgebbar und optional anpassbar. Der Toleranzbereich muss sich nicht notwendig symmetrisch um 100% erstrecken. Im Falle einer Verletzung des Toleranzbereichs wird die Erhaltungsregelung wieder aktiviert. Dies bewirkt im dargestellten Beispiel ein Ansteigen der Sollrate für den $CO_2$-Abbau (Zeitabschnitt II). Die Wiederaktivierung der Erhaltungsregelung endet beim gezeigten Beispiel mit der Rückkehr des Trendparameters etCO2equal in den Toleranzbereich.

Andere Kriterien für die Beendigung der wiederaktivierten Erhaltungsregelung sind alternativ ebenso möglich (siehe oben). Nachdem die Erhaltungsregelung wieder deaktiviert wurde, beginnt erneut das Zurückfahren der Sollrate für den $CO_2$-Abbau ($CO_2$Rt). Das Zurückfahren dauert während des Zeitabschnitts III an und endet, wenn die Sollrate für den $CO_2$-Abbau ($CO_2$Rt) einen vorgegebenen oder vorgebbaren unteren Grenzwert erreicht. Anschließend arbeitet der $CO_2$-Austauscher 16 mit der zuletzt erreichten Sollrate für den $CO_2$-Abbau weiter, bis der $CO_2$-Austauscher 16 von einem Bediener des Systems 10 deaktiviert und entfernt wird.

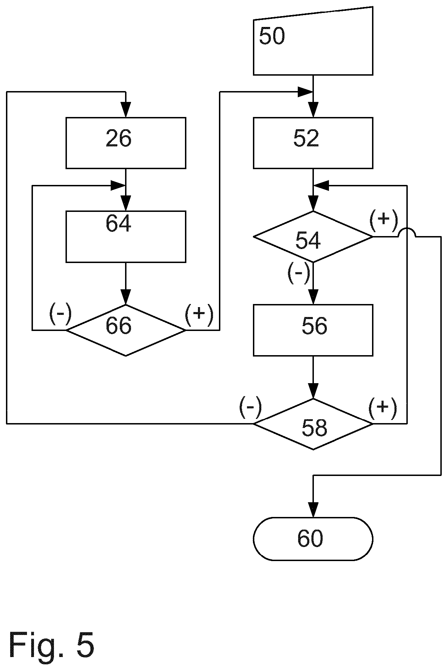

Die Darstellung in Figur 5 veranschaulicht den Entwöhnungsmodus - wie vorstehend beschrieben - anhand eines schematisch vereinfachten Flussdiagramms.

Der Entwöhnungsmodus wird durch eine Bedienhandlung eines Benutzers aktiviert (Block 50). Im Entwöhnungsmodus wird zunächst die Erhaltungsregelung deaktiviert (Block 52). Dann wird geprüft (Block 54), ob eine im Entwöhnungsmodus zurückzufahrende Sollrate für den $CO_2$-Abbau ($CO_2$Rt) einen vorgegebenen oder vorgebbaren unteren Grenzwert bereits erreicht hat. Unmittelbar nach der Aktivierung des Entwöhnungsmodus kann dies normalerweise nicht der Fall sein. Die Bedingung ist also normalerweise nicht erfüllt und entsprechend folgt die Ausführung dem "Minus"-Zweig und anschließend wird die Sollrate für den $CO_2$-Abbau ($CO_2$Rt) reduziert (Block 56). Anschließend wird geprüft (Block 58), ob der Trendparameter noch innerhalb des Toleranzbereichs liegt. Solange dies der Fall ist ("Plus"-Zweig), wird vor den Block 54 verzweigt. Dort wird geprüft, ob der untere Grenzwert für die $CO_2$-Abbau-Sollrate bereits erreicht ist (Block 54), und solange dies nicht der Fall ist, wird die Sollrate reduziert (Block 56) und anschließend der Trendparameter in Bezug auf den Toleranzbereich geprüft (Block 58). Solange der Trendparameter im Toleranzbereich bleibt und der Grenzwert für die $CO_2$-Abbau-Sollrate noch nicht erreicht ist, erfolgt mittels dieser Teilfunktionalität das Zurückfahren der $CO_2$-Abbau-Sollrate, wobei die Darstellung in dem schematisch vereinfachten Flussdiagramm nicht berücksichtigt, dass das Zurückfahren bevorzugt mit einer vorgegebenen Abnahmerate pro Zeit erfolgt, zum Beispiel 3 ml/min pro Stunde. Wenn beim Zurückfahren der $CO_2$-Abbau-Sollrate ermittelt wird (Block 54), dass der Grenzwert für die $CO_2$-Abbau-Sollrate erreicht ist, endet der Entwöhnungsmodus (Block 60) und die $CO_2$-Abbau-Sollrate wird nicht weiter reduziert. Während des Zurückfahrens der $CO_2$-Abbau-Sollrate (Blöcke 54, 56, 58) kann sich allerdings ergeben, dass sich beim Prüfen des Trendparameters in Bezug auf den Toleranzbereich (Block 58) ergibt, dass der Trendparameter den Toleranzbereich verlassen hat. Dann wird ("Minus"-Zweig) die Erhaltungsregelung aktiviert (Block 62) und anschließend die Erhaltungsregelung ausgeführt (Block 64). Während der Wirkung der Erhaltungsregelung wird geprüft (Block 66), ob der Trendparameter einem vorgegebenen oder vorgebbaren Gütekriterium genügt. Als Gütekriterium kann zum Beispiel definiert sein, dass der Trendparameter wieder in den Toleranzbereich zurückgekehrt sein muss, zumindest einmal einen vorgegebenen oder vorgebbaren Wert, zum Beispiel 100%, erreicht haben muss oder dergleichen (siehe oben). Wenn dies der Fall ist ("Plus"-Zweig), wird zur erneuten Deaktivierung der Erhaltungsregelung verzweigt (Block 52) und danach beginnt wieder das Zurückfahren der $CO_2$-Abbau-Sollrate. Solange der Trendparameter dem Gütekriterium nicht genügt ("Minus"-Zweig), wird die Erhaltungsregelung (Block 64) ausgeführt.

Soweit vorstehend vorgegebene oder vorgebbare Werte erwähnt sind, handelt es sich bevorzugt um grundsätzlich veränderliche Daten, die im Speicher 32 der Steuereinheit 22 vorgehalten werden und auf die beim Betrieb des Systems automatisch zugegriffen wird. Vorgegebene Werte werden zum Beispiel bei der Auslieferung oder bei der ersten Verwendung der Steuerungseinheit 22 ausgewählt und in den Speicher 32 geladen. Vorgebbare Werte sind Werte, die zum Beispiel auch während des Betriebs der Steuerungseinheit 22 oder zwischen aufeinanderfolgenden Einsätzen der Steuerungseinheit 22 durch einen Bediener der Steuerungseinheit 22 oder einen Bediener des Systems 10, insbesondere einen Arzt, im Sinne einer Parametrierung veränderbar sind.

BEZUGSZEICHENLISTE

| | |
|---|---|
| 10 | System |
| 12 | Patient |
| 14 | Medizingerät, Beatmungsgerät |
| 16 | Medizingerät, $CO_2$-Austauscher |
| 18 | Atemmaske |
| 20 | Sensorik |
| 22 | Medizingerät, Steuerungseinheit |
| 24 | Anzeigeeinheit |
| 26 | Eingabevorrichtung |
| 30 | Verarbeitungseinheit |
| 32 | Speicher |
| 34 | Steuerungsprogramm |
| 36 | Startwertspeicherstelle |
| 40 | Regelkreis |
| 42 | Regler |
| 44 | Startwert |
| 46 | Normierer |
| 50-66 | Block (im Flussdiagramm) |

**Patentansprüche**

1. System (10) zur Unterstützung des Blutgasaustauschs eines Patienten (12) mittels Beatmung einerseits sowie mittels extrakorporalem Blutgasaustausch mittels eines $CO_2$-Austauschers (16) andererseits,

   wobei das System (10) zur Beatmung ein Medizingerät in Form eines Beatmungsgeräts (14) sowie zum extrakorporalen Blutgasaustausch ein Medizingerät in Form eines $CO_2$-Austauschers (16) umfasst,
   wobei mittels einer Sensorik (20) ein Messwert bezüglich einer exspiratorischen, $CO_2$-Konzentration im Atemgas des Patienten (12) erfassbar ist,
   **dadurch gekennzeichnet, dass**
   mittels einer Bedienhandlung am System (10) ein aktueller Messwert als Startwert auswählbar ist, festgehalten wird und als Basis für eine Regelung mittels eines Reglers (42) fungiert und
   wobei dem Regler (42) eine Differenz aus einem Trendparameter und einem Sollwert für den Trendparameter dem Regler (42) zuführbar ist,
   wobei der Trendparameter als ein Quotient aus einem jeweils aktuell ermittelten Messwert und dem Startwert gebildet wird und wobei der Regler (42) auf den CO2-Austauscher (16) wirkt.

2. System (10) nach Anspruch 1,
   wobei mittels der Sensorik (20) als Messwert bezüglich der exspiratorischen, $CO_2$-Konzentration im Atemgas des Patienten (12) ein Messwert bezüglich einer endexspiratorischen $CO_2$-Konzentration (et $CO_2$) im Atemgas des Patienten (12) erfassbar ist.

3. System (10) nach Anspruch 1 oder nach Anspruch 2,

   wobei mit dem Startwert und einem jeweils aktuell ermittelten Messwert der Trendparameter ermittelbar ist und
   wobei eine Differenz aus einem Sollwert für den Trendparameter und einem jeweils aktuellen Wert des Trendparameters dem Regler (42) zuführbar ist, welcher auf den $CO_2$-Austauscher (16) wirkt.

4. System (10) nach Anspruch 3,
   wobei der Trendparameter in Form einer Normierung des jeweils aktuell ermittelten Messwerts in Relation zu dem Startwert ermittelbar ist.

5. System (10) nach einem der vorangehenden Ansprüche, wobei der jeweils aktuelle Messwert an einer Anzeigeeinheit (24) ausgebbar ist und mittels der Bedienhandlung der angezeigte Messwert als Startwert auswählbar ist.

6. System (10) nach einem der vorangehenden Ansprüche, wobei mittels einer Sensorik (20) eine Spontanatemfrequenz des Patienten (12) überwachbar ist und im Falle einer Überschreitung eines vorgegebenen oder vorgebbaren Schwellwerts ein Signalelement ansteuerbar ist.

7. System (10) nach einem der vorangehenden Ansprüche,

wobei mittels einer Bedienhandlung am System (10) ein Entwöhnungsmodus aktivierbar ist, wobei im Entwöhnungsmodus eine $CO_2$-Abbau-Sollrate automatisch und gesteuert reduzierbar ist.

8. System (10) nach Anspruch 7,

wobei beim Beginn der Reduktion der $CO_2$-Abbau-Sollrate ein Einfluss des Reglers (42) auf den $CO_2$-Austauscher (16) deaktivierbar ist, wobei während der Reduktion der $CO_2$-Abbau-Sollrate der Trendparameter in Bezug auf einen vorgegebenen oder vorgebbaren Toleranzbereich überwachbar ist, wobei bei einer Verletzung des Toleranzbereichs einerseits der Einfluss des Reglers (42) auf den $CO_2$-Austauscher (16) wieder aktivierbar und andererseits die Reduktion der $CO_2$-Abbau-Sollrate deaktivierbar ist.

9. Steuerungsprogramm (34) in Form eines Computerprogramms mit Programmcodemitteln, um beim Betrieb eines Systems (10) nach einem der vorangehenden Ansprüche die folgenden Verfahrensschritte auszuführen, wenn das Steuerungsprogramm (34) mittels einer Verarbeitungseinheit (30) eines Medizingeräts (14, 16, 22) ausgeführt wird:

• Erfassen eines Messwerts bezüglich einer exspiratorischen, insbesondere endexspiratorischen $CO_2$-Konzentration (et$CO_2$) im Atemgas eines Patienten (12) mittels der Sensorik (20); • Festhalten eines mittels einer Bedienhandlung am System (10) als Startwert ausgewählten aktuellen Messwerts, • Zuführen einer Differenz aus einem Trendparameter und einem Sollwert für den Trendparameter,

wobei der Trendparameter als ein Quotient aus einem jeweils aktuell ermittelten Messwert und dem Startwert gebildet wird.

## Claims

1. System (10) for supporting the blood gas exchange of a patient (12) by means of both ventilation and extracorporeal blood gas exchange by means of a $CO_2$-exchanger (16),

the system (10) comprising a medical device in the form of a ventilator (14) for ventilation and a medical device in the form of a $CO_2$ exchanger (16) for extracorporeal blood gas exchange, it being possible to detect a measured value relating to an expiratory $CO_2$ concentration in the patient's (12) respiratory gas by means of a sensor system (20), **characterized in that,** by means of an operating action on the system (10), a current measured value can be selected as a starting value, is recorded, and functions as a basis for control by means of a controller (42) and it being possible to supply, to the controller (42), a difference between a trend parameter and a target value for the trend parameter, to the controller (42), the trend parameter being formed as a quotient of a currently determined measured value and the starting value, and the controller (42) acting on the CO2 exchanger (16).

2. System (10) according to claim 1, wherein a measured value relating to an end-expiratory $CO_2$ concentration (et $CO_2$) can be detected in the patient's (12) respiratory gas by means of the sensor system (20) as a measured value relating to the expiratory $CO_2$ concentration in the patient's (12) respiratory gas.

**3.** System (10) according to claim 1 or claim 2,

wherein the trend parameter can be determined using the starting value and a currently determined measured value and

wherein a difference between a target value for the trend parameter and a current value of the trend parameter can be supplied to the controller (42), which acts on the $CO_2$ exchanger (16).

**4.** System (10) according to claim 3,
wherein the trend parameter can be determined in the form of a normalization of the currently determined measured value in relation to the starting value.

**5.** System (10) according to any of the preceding claims,
wherein the current measured value can be output on a display unit (24) and the displayed measured value can be selected as the starting value by means of the operating action.

**6.** System (10) according to any of the preceding claims,
wherein a spontaneous respiratory frequency of the patient (12) can be monitored by means of a sensor system (20) and a signal element can be activated if a predetermined or predeterminable threshold value is exceeded.

**7.** System (10) according to any of the preceding claims,

wherein a weaning mode can be activated by means of an operating action on the system (10),
wherein, in the weaning mode, a $CO_2$ degradation target rate can be reduced automatically and in a controlled manner.

**8.** System (10) according to claim 7,

wherein, at the beginning of the reduction of the $CO_2$ degradation target rate, an influence of the controller (42) on the $CO_2$ exchanger (16) can be deactivated,
wherein, during the reduction of $CO_2$ degradation target rate, the trend parameter can be monitored in relation to a predetermined or predeterminable tolerance range,
wherein, in the event of a violation of the tolerance range, the influence of the controller (42) on the $CO_2$ exchanger (16) can be reactivated and the reduction of $CO_2$ degradation target rate can be deactivated.

**9.** Control program (34) in the form of a computer program having program code means for carrying out the following method steps during operation of a system (10) according to any of the preceding claims, when the control program (34) is executed by means of a processing unit (30) of a medical device (14, 16, 22):

• detecting a measured value relating to an expiratory, particularly end-expiratory, $CO_2$ concentration ($etCO_2$) in the respiratory gas of a patient (12) by means of the sensor system (20);
• recording a current measured value selected as a starting value by means of an operating action on the system (10),
• supplying a difference between a trend parameter and a target value for the trend parameter,

wherein the trend parameter is formed as a quotient of a currently determined measured value and the starting value.

**Revendications**

**1.** Système (10) permettant d'assister l'échange de gaz du sang d'un patient (12) au moyen d'une ventilation d'une part et au moyen d'un échange extracorporel de gaz du sang au moyen d'un échangeur de $CO_2$ (16) d'autre part,

dans lequel le système (10) comprend un appareil médical sous la forme d'un respirateur (14) pour la ventilation ainsi qu'un appareil médical sous la forme d'un échangeur de $CO_2$ (16) pour l'échange extracorporel de gaz du sang,
dans lequel une valeur de mesure concernant une concentration expiratoire de $CO_2$ dans le gaz respiratoire du patient (12) peut être détectée au moyen d'un système de capteurs (20),
**caractérisé en ce que,** au moyen d'une action de commande sur le système (10), une valeur de mesure actuelle peut être sélectionnée comme valeur de départ, est maintenue et sert de base pour une régulation au moyen d'un régulateur (42) et

dans lequel, au régulateur (42), une différence entre un paramètre de tendance et une valeur de consigne pour le paramètre de tendance peut être fournie au régulateur (42),

dans lequel le paramètre de tendance est formé comme un quotient d'une valeur de mesure respectivement déterminée actuellement et de la valeur de départ et dans lequel le régulateur (42) agit sur l'échangeur de $CO_2$ (16).

2. Système (10) selon la revendication 1,
   dans lequel une valeur de mesure concernant une concentration de $CO_2$ en fin d'expiration (et $CO_2$) dans le gaz respiratoire du patient (12) peut être détectée comme valeur de mesure concernant la concentration de $CO_2$ expiratoire dans le gaz respiratoire du patient (12) au moyen du système de capteurs (20).

3. Système (10) selon la revendication 1 ou la revendication 2,

   dans lequel le paramètre de tendance peut être déterminé avec la valeur de départ et une valeur de mesure respectivement déterminée actuellement et
   dans lequel une différence entre une valeur de consigne pour le paramètre de tendance et une valeur respectivement actuelle du paramètre de tendance peut être fournie au régulateur (42) qui agit sur l'échangeur de $CO_2$ (16).

4. Système (10) selon la revendication 3,
   dans lequel le paramètre de tendance peut être déterminé sous la forme d'une normalisation de la valeur de mesure respectivement déterminée actuellement par rapport à la valeur de départ.

5. Système (10) selon l'une des revendications précédentes,
   dans lequel la valeur de mesure respectivement actuelle peut être sortie sur une unité d'affichage (24) et la valeur de mesure affichée peut être sélectionnée comme valeur de départ au moyen de l'action de commande.

6. Système (10) selon l'une des revendications précédentes,
   dans lequel une fréquence de respiration spontanée du patient (12) peut être surveillée au moyen d'un système de capteurs (20) et un élément de signalisation peut être commandé en cas de dépassement d'une valeur seuil prédéfinie ou pouvant être prédéfinie.

7. Système (10) selon l'une des revendications précédentes,

   dans lequel un mode de désintoxication peut être activé au moyen d'une action de commande sur le système (10),
   dans lequel un taux de consigne d'élimination du $CO_2$ peut être réduit automatiquement et de manière contrôlée dans le mode de désintoxication.

8. Système (10) selon la revendication 7,

   dans lequel une influence du régulateur (42) sur l'échangeur de $CO_2$ (16) peut être désactivée au début de la réduction du taux de consigne d'élimination du $CO_2$,
   dans lequel le paramètre de tendance peut être surveillé par rapport à une plage de tolérance prédéfinie ou pouvant être prédéfinie pendant la réduction du taux de consigne d'élimination du $CO_2$,
   dans lequel, en cas de violation de la plage de tolérance, d'une part l'influence du régulateur (42) sur l'échangeur de $CO_2$ (16) peut être réactivée, et d'autre part la réduction du taux de consigne d'élimination du $CO_2$ peut être désactivée.

9. Programme de commande (34) sous la forme d'un programme informatique comportant des moyens de code de programme pour exécuter les étapes de procédé suivantes lors du fonctionnement d'un système (10) selon l'une des revendications précédentes, lorsque le programme de commande (34) est exécuté au moyen d'une unité de traitement (30) d'un appareil médical (14, 16, 22) :

   • détection d'une valeur de mesure concernant une concentration de $CO_2$ expiratoire, en particulier en fin d'expiration (et $CO_2$), dans le gaz respiratoire d'un patient (12) au moyen du système de capteurs (20) ;

• maintien d'une valeur de mesure actuelle sélectionnée comme valeur de départ au moyen d'une action de commande sur le système (10),
• fourniture d'une différence entre un paramètre de tendance et une valeur de consigne pour le paramètre de tendance,

dans lequel le paramètre de tendance est formé comme un quotient d'une valeur de mesure respectivement déterminée actuellement et de la valeur de départ.

CO$_2$mess; ETCO$_2$mess

12

18

20

24

14

16

26

10

Fig. 1

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2015034082 A1
- EP 3291854 A1